# EUROPEAN PATENT APPLICATION

(11) **EP 3 903 833 A2**
(43) Date of publication of application: **03.11.2021**
(21) Application number: 21170050.5
(22) Date of filing: 23.04.2021
(51) Int. Cl.: A61L 2/00, A61L 2/26

(54) **STERILIZING BRACELET ASSEMBLY**

(30) Priority: 27.04.2020 TW 109114078; 13.09.2020 US 202017019348
(71) Applicant: Wang, Tse-Chuan, Luadong Township 265 (TW)
(72) Inventor: Wang, Tse-Chuan, 265 Luadong Township (TW); Wang, Ching-Chieh, 265 Luadong Township (TW)
(74) Representative: Straus, Alexander

(57) **Abstract**

The present invention provides a sterilizing bracelet assembly, which can be filled out with sterilizing liquid. The sterilizing bracelet assembly has a housing and a switch; the housing has at least a first accommodation room that is to accommodate liquid, and the switch is connected to the housing and has a nozzle; wherein some of the sterilizing liquid is ejected from the nozzle after the switch is squeezed.

## Description

### BACKGROUND OF THE INVENTION

### Field of the Invention

The present invention generally relates to a bracelet assembly, more particularly to the bracelet assembly that can be filled out with sterilizing liquid, such as alcohol, soap, etc.

### Description of the Prior Art

During the period of the COVID-19, the first thing to fight against the pandemic is to "wash hands frequently" and keep an eye on personal hygiene in order to stay away from the disease. Hence, modern people going outside may carry small types of cleaning products, such as facial cleanser, hand cleanser, dry cleaning hand agent, etc., to clean hands any time after touching external things or other people 's objects.

Therefore, how to design a sterilizing product that is convenient to carry is worth considering for those people who are skilled in the art.

### SUMMARY OF THE INVENTION

The main objective of the present invention provides a bracelet assembly, which is very convenient to wrap around the wrist of a human being. So it is good for modern people to go out and carry and clean their hands.

The bracelet assembly has a housing and a switch; the housing has at least a first accommodation room that is to accommodate sterilizing liquid, and the switch is connected to the housing and has a nozzle; wherein some of the sterilizing liquid is ejected from the nozzle after the switch is squeezed.

The sterilizing bracelet assembly further has an electric ring. The electric ring has a touch switch and a first Bluetooth module. The first Bluetooth module sends an activating signal after the touch switch is touched. The switch has a second Bluetooth module that is to receive the activating signal, some of the sterilizing liquid is ejected out from the nozzle after the activating signal is received.

The sterilizing bracelet assembly further has a smart watch. The smart watch has a first Bluetooth module. The first Bluetooth module sends an activating signal after the smart watch receives a sound signal. The switch has a second Bluetooth module that is to receive the activating signal. Some of the sterilizing liquid is ejected out from the nozzle after the activating signal is received.

For aforesaid sterilizing bracelet assembly, the smart watch further has a display screen for displaying a remaining amount of the sterilizing liquid.

For aforesaid sterilizing bracelet assembly, the switch further has a valve. The valve is between the first accommodation room and the nozzle. The valve is at an open state when the switch is vertical to the housing, and the valve is at a close state when the switch is parallel to the housing.

For aforesaid sterilizing bracelet assembly, the number of the switch is multiple, and the number of the first accommodation room is multiple as well. Each of the switches is corresponding to each of the first accommodation rooms.

For aforesaid sterilizing bracelet assembly, there are two magnets. One of the magnets is disposed at one end of the housing, and another is at another end of the housing.

For aforesaid sterilizing bracelet assembly, the sterilizing liquid is injected into the first accommodation room from an external supplement machine.

For another embodiment of the present invention, the sterilizing bracelet assembly has a housing, a supplement box and two switches. The housing has two first accommodation rooms that are to accommodate sterilizing liquid. The supplement box has two second accommodation rooms that are to accommodate the liquid. The two switches are connected to the supplement box. Each of the two switches has a nozzle and is corresponding to each of the two second accommodation rooms. In addition, one of the first accommodation rooms is connected to one of the second accommodation rooms, and the other first accommodation room is connected to the other second accommodation rooms. Some of the sterilizing liquid is ejected from the nozzle after the switch is squeezed.

For aforesaid sterilizing bracelet assembly, the switch is a pneumatic switch or an electronic switch.

For another embodiment of the present invention, the bracelet assembly has a plurality of housings and a connecting line. The housing has a through hole and a first accommodation room. The first accommodation room is to accommodate sterilizing liquid. Besides, the connecting line is connected to each of the housings in series by means of the through holes.

For aforesaid sterilizing bracelet assembly, the housing further has a nozzle and a cover. The nozzle is disposed on a surface of the housing. The cover blocks the nozzle. Some of the sterilizing liquid is ejected from the nozzle after the housing is squeezed.

Other and further features, advantages, and benefits of the invention will become apparent in the following description taken in conjunction with the following drawings. It is to be understood that the foregoing general description and following detailed description are exemplary and explanatory but are not to be restrictive of the invention. The accompanying drawings are incorporated in and constitute a part of this application and, together with the description, serve to explain the principles of the invention in general terms. Like numerals refer to like parts throughout the disclosure.

### BRIEF DESCRIPTION OF THE DRAWINGS

The objects, spirits, and advantages of the preferred embodiments of the present invention will be readily understood by the accompanying drawings and detailed descriptions, wherein:
Fig. 1A illustrates a schematic block view of a sterilizing bracelet assembly of a first preferred embodiment of the present invention;
Fig. 1B illustrates a perspective view of the sterilizing bracelet assembly of the present invention;
Fig. 1C illustrates a perspective view of another aspect of the sterilizing bracelet assembly of the present invention;
Fig. 2A illustrates a schematic view of a switch vertical to a housing of the present invention;
Fig. 2B illustrates a schematic view of the switch parallel to the housing of the present invention;
Fig. 3 illustrates a schematic view of the sterilizing bracelet assembly wrapping around a wrist of the present invention;
Fig. 4A illustrates a schematic view of a sterilizing bracelet assembly of a second preferred embodiment of the present invention;
Fig. 4B illustrates a schematic view of the sterilizing bracelet assembly with a ring type of the present invention;
Fig. 5 illustrates a schematic view of liquid injected into a first accommodation room from a supplement machine of the present invention;
Fig. 6 illustrates a perspective view of a sterilizing bracelet assembly of a third preferred embodiment of the present invention;
Fig. 7A illustrates a schematic block view of a sterilizing bracelet assembly of a fourth preferred embodiment of the present invention;
Fig. 7B illustrates a perspective view of an electric ring and the housing wearing on a hand of the present invention;
Fig. 8A illustrates a schematic block view of a sterilizing bracelet assembly of a fifth preferred embodiment of the present invention;
Fig. 8B illustrates a perspective view of the sterilizing bracelet assembly of the present invention;
Fig. 9A illustrates a perspective view of a sterilizing bracelet assembly of a sixth preferred embodiment of the present invention;
Fig. 9B illustrates a perspective view prior to assembling a supplement box and a housing;
Fig. 10A illustrates a perspective view of a sterilizing bracelet assembly of a seventh preferred embodiment of the present invention;
Fig. 10B illustrates a schematic view of a housing under different applications;
Fig. 10C illustrates a perspective view of another aspect of the sterilizing bracelet assembly of the present invention;
Fig. 11 illustrates a perspective view of another aspect of the sterilizing bracelet assembly of the present invention;
Fig. 12 illustrates a perspective view of another aspect of the sterilizing bracelet assembly of the present invention;
Fig. 13 and Fig. 14 illustrate schematic views of a housing under different applications;
Fig. 15 illustrates a perspective view of a sterilizing bracelet assembly of an eighth preferred embodiment of the present invention;
Fig. 16 illustrates a schematic view of a sterilizing bracelet assembly of a nineth preferred embodiment of the present invention;
Fig. 17 illustrates a perspective view of the sterilizing bracelet assembly under an application;
Fig. 18 illustrates a schematic view of a sterilizing bracelet assembly of a nineth preferred embodiment of the present invention; and
Fig. 19 illustrates a perspective view of the sterilizing bracelet assembly under an application.

### DETAILED DESCRIPTION OF THE INVENTION

Following preferred embodiments and figures will be described in detail so as to achieve aforesaid objects.

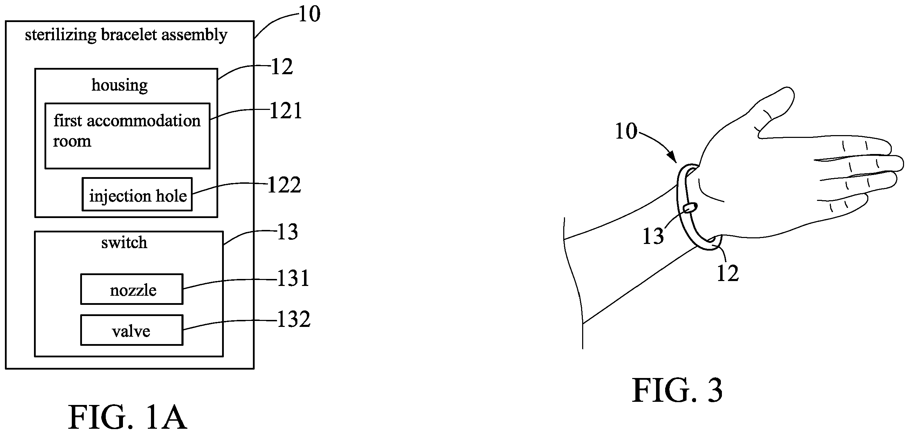

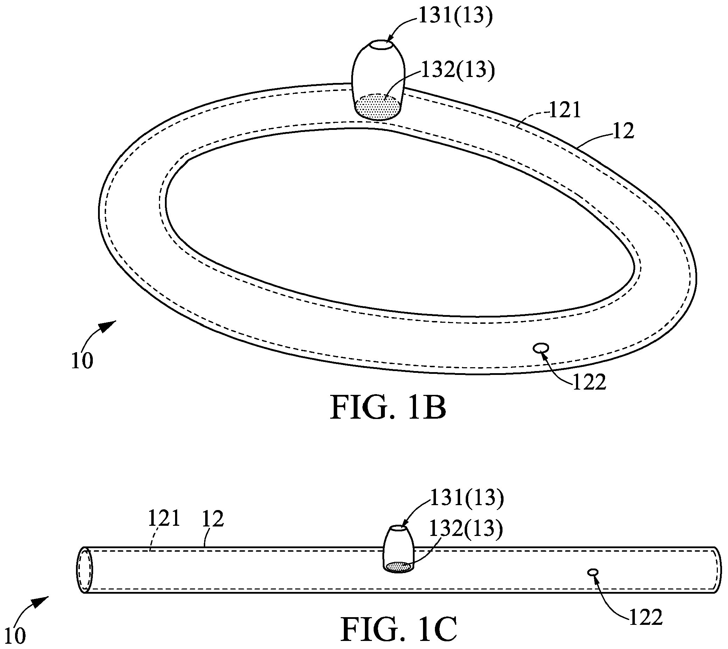

Please refer to Fig. 1A, Fig. 1B and Fig. 1C, which illustrate a schematic block view of a sterilizing bracelet assembly 10 of a first preferred embodiment of the present invention, a perspective view of the sterilizing bracelet assembly 10 of the present invention, and a perspective view of another aspect of the sterilizing bracelet assembly 10 of the present invention. The sterilizing bracelet assembly 10 includes a housing 12 and a switch 13, and the switch 13 can be a pneumatic switch or an electronic switch. In addition, the housing 12 is connected to the switch 13, and can be made of silicon or rubber in order to maintain flexibility. The housing 12 can be shaped as a ring or linear type, and has at least one injection hole 122. Liquid is introduced into a first accommodation room 121 by means of the injection hole 122, but the injection hole 122 is not a necessary part.

The housing 12 has the first accommodation room 121 in order to accommodate the liquid, such as alcohol, dry-cleaning hand lotion or hand lotion. The switch 13 has a nozzle 131 and a valve 132, wherein the valve 132 is between the first accommodation room 121 and the nozzle 131.

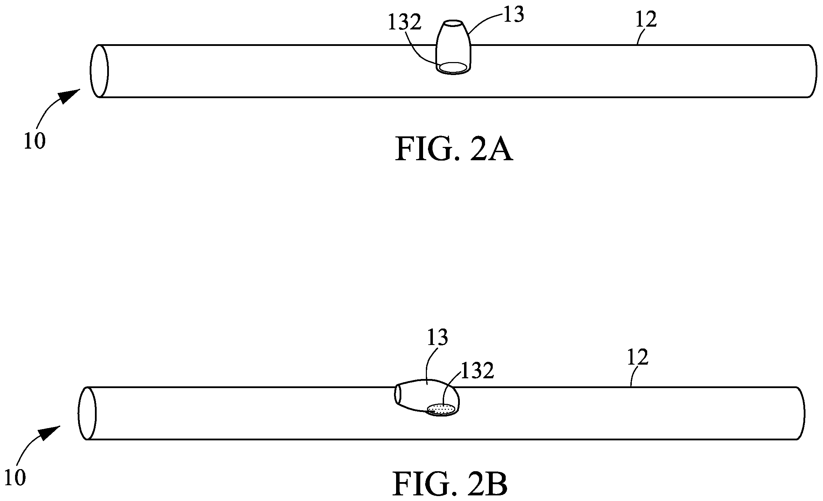

Please refer to Fig. 2A and Fig. 2B, which illustrate a schematic view of the switch 13 vertical to the housing 12 of the present invention and a schematic view of the switch 13 parallel to the housing 12 of the present invention. A valve 132 is at an open state when the switch 13 is vertical to the housing 12, on the other hand, the valve 132 is at a close state when the switch 13 is parallel to the housing 12. Therefore, the liquid could be kept in the first accommodation room 121, and the valve 132 is opened when cleaning is a must.

With reference to Fig. 3, which illustrates a schematic view of the sterilizing bracelet assembly 10 wrapping around a wrist of the present invention. Compared to small types of cleaning products, the ring type of sterilizing bracelet assembly 10 is more convenient to wear on the wrist of a human being, and therefore it is good to be carried on.

The nozzle 131 may eject some of the liquid when pressing the switch 13. As a matter of fact, pressing the switch 13 causes that the compressed air forces the liquid in the first accommodation room 121 to be ejected out from one end of the first accommodation room 121 to the other. For example, the liquid as alcohol or dry-cleaning hand lotion may be ejected to the hand of a human being, and the amount of ejection is about the volume of the switch 13. If the volume is 1 c.c., each ejection will be around 1 c.c. In other words, a user with the sterilizing bracelet assembly 10 may clean his hands simultaneously when touching external things.

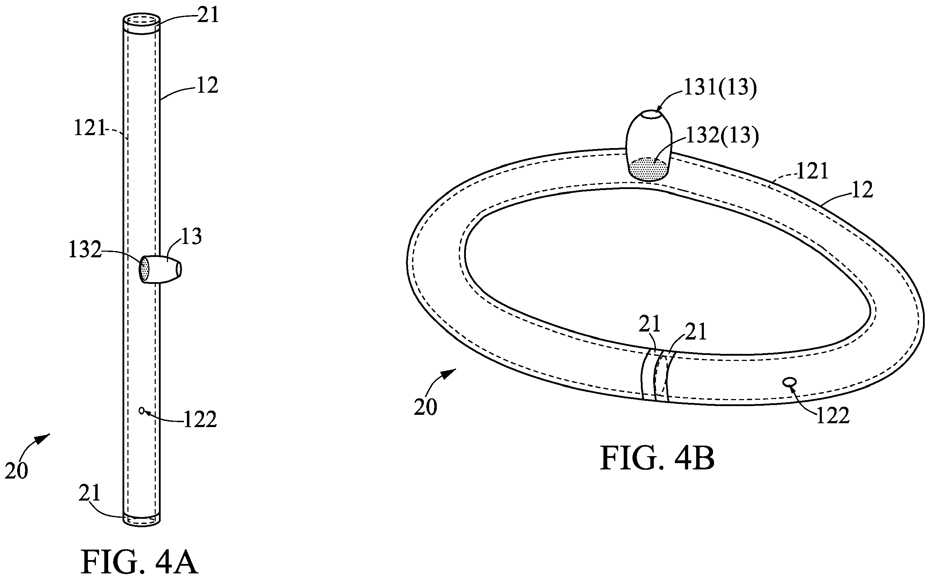

With reference to Fig. 4A and Fig. 4B, which illustrate a schematic view of a sterilizing bracelet assembly 20 of a second preferred embodiment of the present invention and a schematic view of the sterilizing bracelet assembly 20 with a ring type of the present invention. The differences between the sterilizing bracelet assembly 20 and the sterilizing bracelet assembly 10 will be mentioned as following. The sterilizing bracelet assembly 20 further has two magnets 21, one of the magnets 21 is disposed at one end of the housing 12, another magnet 21 is at another end. Such design makes the sterilizing bracelet assembly 20 be a ring type because of the magnetic forces of the two magnets 21, so as to conveniently wear on or take off from the wrist.

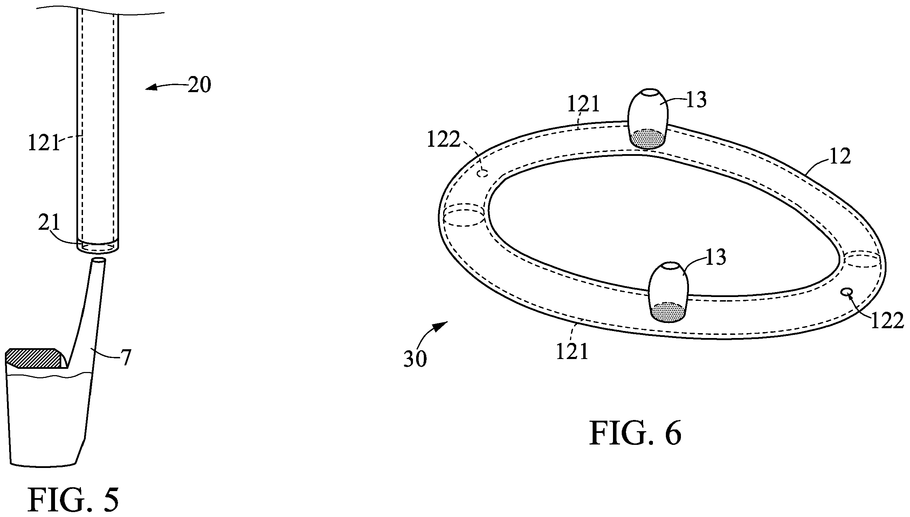

Please refer to Fig. 5, which illustrates a schematic view of the liquid injected into the first accommodation room 121 from a supplement machine 7 of the present invention. There are two ways to induce the liquid into the first accommodation room 121, one is through the injection hole 122, and the other is by means of the external supplement machine 7. More specifically, the liquid in the supplement machine 7 can be injected into the first accommodation room 121 from one end of the sterilizing bracelet assembly 20 when the two ends of the sterilizing bracelet assembly 20 are separated. It would be faster and more convenient to introduce the liquid into the sterilizing bracelet assembly 20.

With reference to Fig. 6, which illustrates a perspective view of a sterilizing bracelet assembly 30 of a third preferred embodiment of the present invention. The differences between the sterilizing bracelet assembly 30 and the sterilizing bracelet assembly 10 will be mentioned as following. The number of the switch 13 of the sterilizing bracelet assembly 30 is multiple. As shown in Fig. 6, there are two switches 13. The number of the first accommodation room 121 is multiple as well. That is, the housing 12 for the third preferred embodiment has several first accommodation rooms 121. As shown in Fig. 6, there are two first accommodation rooms 121. Each of the switches 13 is corresponding to each of the first accommodation rooms 121. Therefore, the feature of the design brings that of different first accommodation rooms 121 may contain different liquids, such as dry-cleaning hand lotion and hand lotion. The flexible usage lets a user decide different ways to clean, either dry or wet hand washing.

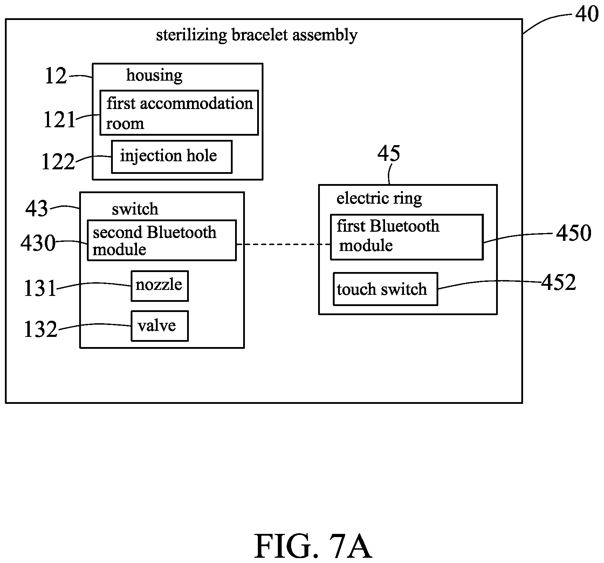

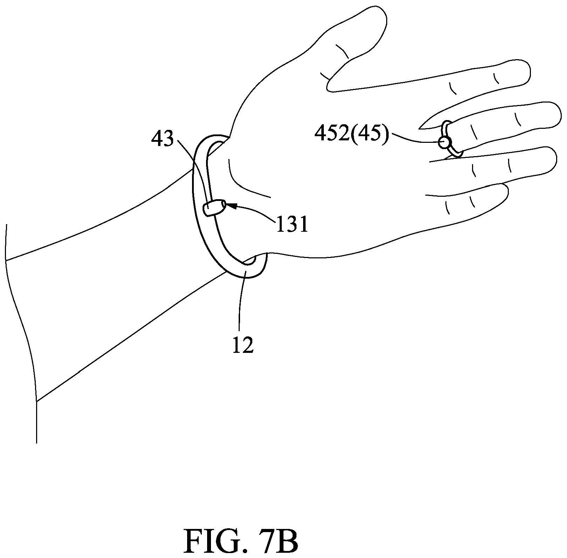

Please refer to Fig. 7A and Fig. 7B, which illustrate a schematic block view of a sterilizing bracelet assembly 40 of a fourth preferred embodiment of the present invention and a perspective view of an electric ring 45 and the housing 12 wearing on a hand of the present invention. The differences between the sterilizing bracelet assembly 40 and the sterilizing bracelet assembly 10 will be mentioned as following. The sterilizing bracelet assembly 40 further has the electric ring 45 that is on a finger and has a touch switch 452 and a first Bluetooth module 450. A switch 43 of the sterilizing bracelet assembly 40 further has a second Bluetooth module 430. The first Bluetooth module 450 sends an activation signal to the second Bluetooth module 430 after the touch switch 452 is touched. Continuously, some of the liquid is ejected out from the nozzle 131 of the switch 43 after the activating signal is received by the second Bluetooth module 430. As it can be seen, the fourth preferred embodiment would be faster and more convenient to let a user obtain the liquid for cleaning.

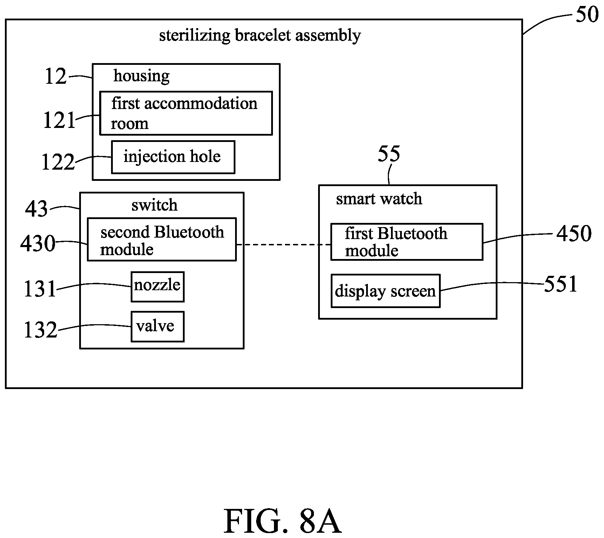

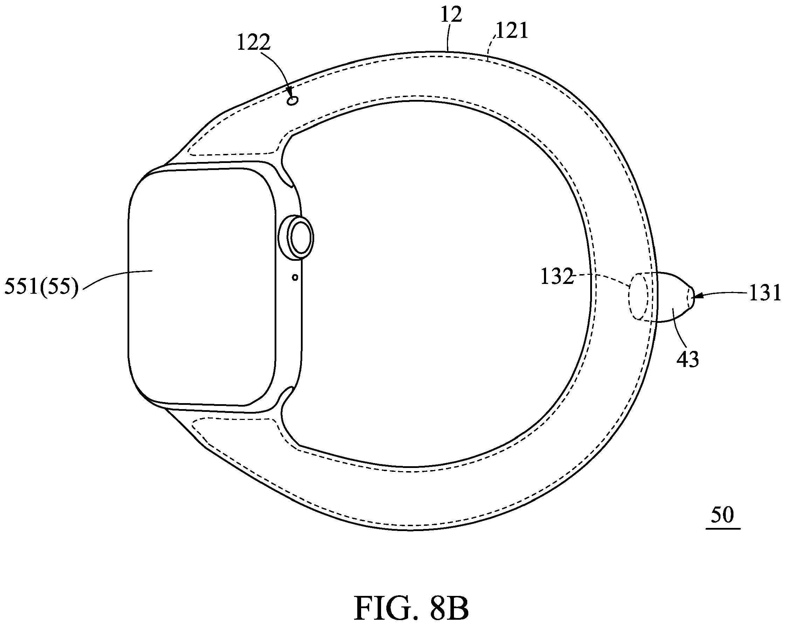

With reference to Fig. 8A and Fig. 8B, which illustrate a schematic block view of a sterilizing bracelet assembly 50 of a fifth preferred embodiment of the present invention and a perspective view of the sterilizing bracelet assembly 50 of the present invention. The differences between the sterilizing bracelet assembly 50 and the sterilizing bracelet assembly 10 will be mentioned as following. The sterilizing bracelet assembly 50 further has a smart watch 55 as Apple watch, wherein the smart watch 55 has a display screen 551 and the first Bluetooth module 450. The switch 43 of the sterilizing bracelet assembly 50 further has the second Bluetooth module 430. Obviously, as shown in Fig. 8B, the housing 12 is the belt of the smart watch 55, so that the user is able to wear the sterilizing bracelet assembly 50.

As soon as the smart watch receives a sound signal sent from the user, the first Bluetooth module 450 sends an activating signal to the second Bluetooth module 430. Continuously, some of the liquid is ejected out from the nozzle 131 of the switch 43 after the second Bluetooth module 430 receives the activating signal, so as to let the user clean his hands in time. As aforesaid, once ejecting the liquid, the display screen 551 of the smart watch 55 displays a remaining amount of the liquid. Moreover, each ejection amount, such as 1 or 3 c.c., can be adjusted by the smart watch 55.

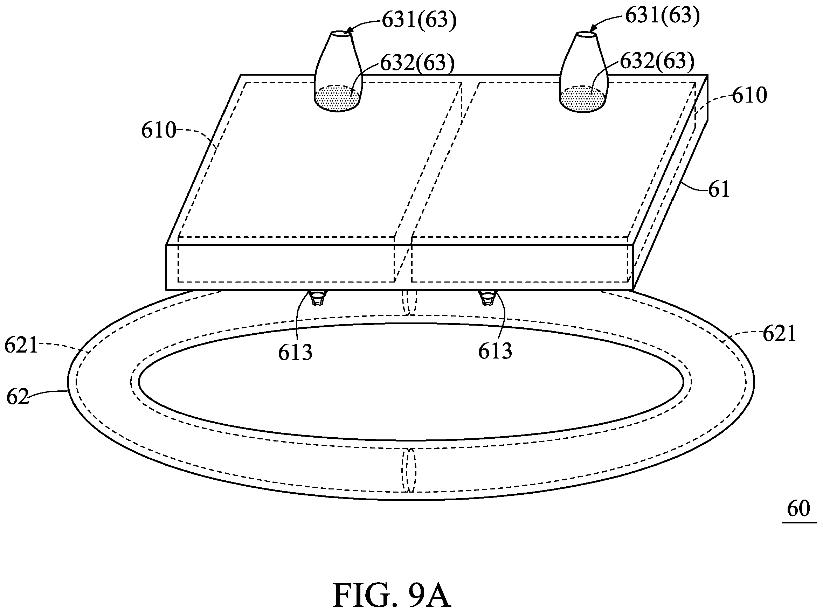

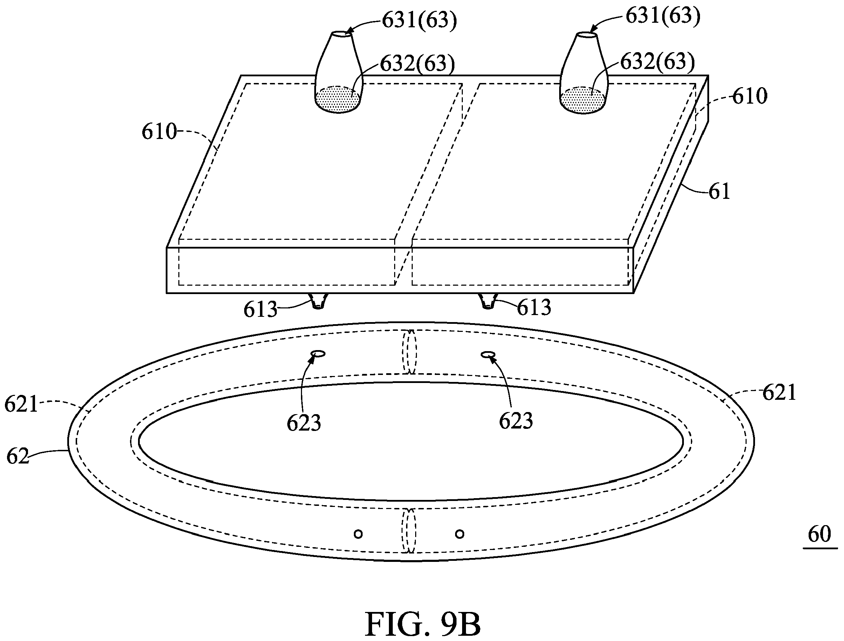

Referring to Fig. 9A and Fig. 9B, which illustrate a perspective view of a sterilizing bracelet assembly 60 of a sixth preferred embodiment of the present invention and a perspective view prior to assembling a supplement box 61 and a housing 62. The sterilizing bracelet assembly 60 has the housing 62, the supplement box 61 and two switches 63, wherein the supplement box 61 is detachably assembled to the housing 62. More specifically, the supplement box 61 has two connecting tube 613, and the housing 62 has two insertion holes 623. The connecting tube 613 embeds in the insertion holes 623 in order to install the supplement box 61 on the housing 62.

Besides, the housing 62 has two first accommodation rooms 621 that are to accommodate liquid. The supplement box 61 has two second accommodation rooms 610 that are to accommodate the liquid. The two switches 63 are connected to the supplement box 61, each of the two switches 63 has a nozzle 631 and a valve 632 and is corresponding to each of the two second accommodation room 610. The valve 632 is between the second accommodation room 610 and the nozzle 631.

One of the first accommodation rooms 621 is connected to one of the second accommodation rooms 610, and the other first accommodation rooms 621 is connected to the other second accommodation rooms 610. Hence, the first accommodation rooms 621 and the second accommodation rooms 610, which connect to each other, contain the same liquid, such as dry-cleaning hand lotion. Another pair of the first accommodation rooms 621 and the second accommodation rooms 610 will be the same function for storing hand lotion. Again, as aforesaid, the nozzle 631 sprays some liquid when the switch 63 is squeezed. In addition, the supplement box 61 functions to increase a storing amount of the liquid, so that the sterilizing bracelet assembly 60 is available to a user who often cleans hands.

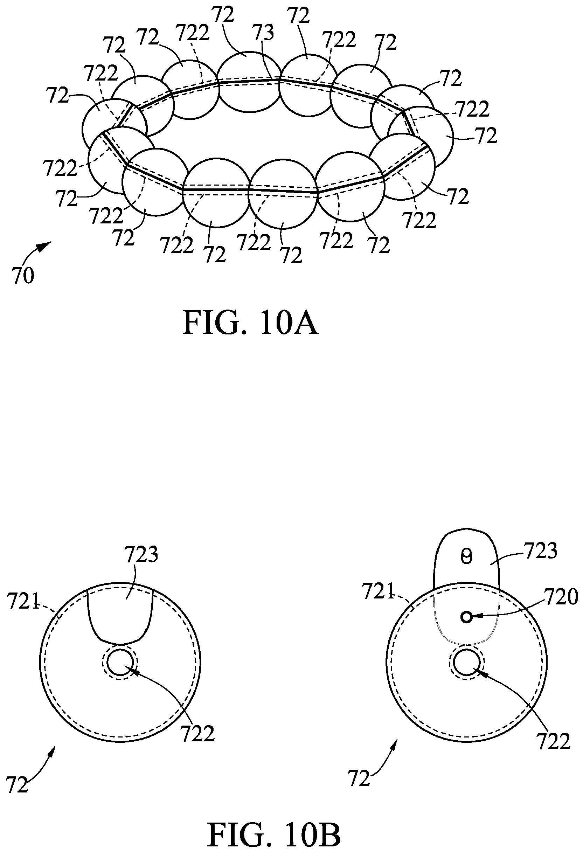

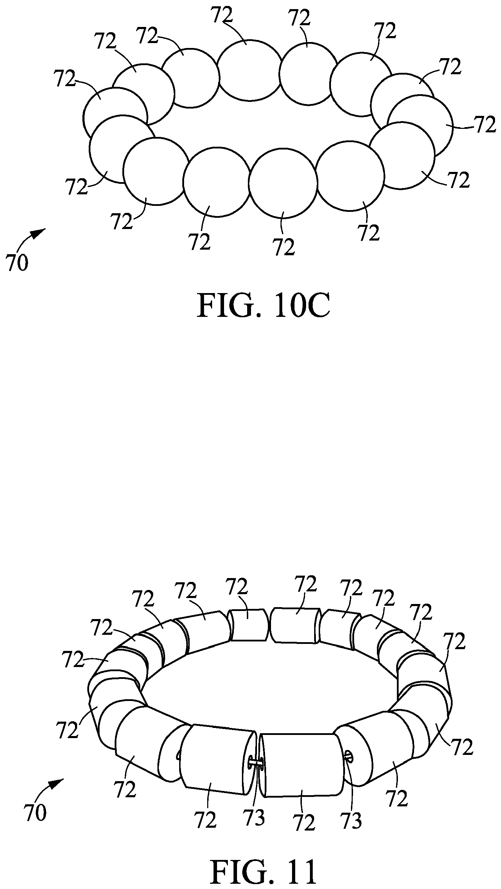

With reference to Fig. 10A, Fig. 10B and Fig. 10C, which illustrate a perspective view of a sterilizing bracelet assembly 70 of a seventh preferred embodiment of the present invention, a schematic view of a housing 72 under different applications, and a perspective view of another aspect of the sterilizing bracelet assembly 70 of the present invention. The sterilizing bracelet assembly 70 has a plurality of housings 72 and a connecting line 73. The housing 72 has a nozzle 720, a cover 723, a through hole 722, and a first accommodation room 721. The connecting line 73 is connected to each of the housings 72 in series by means of the through holes 722. The first accommodation room 721 is to accommodate liquid. The nozzle 720 and the cover 723 are on a surface of the housing 72 in order to seal the nozzle 720 for preventing liquid leakage. For the preferred embodiment, the connecting line 73 may be flexible. It is not necessary to connect the housings 72 by the connecting line 73. As shown in Fig. 10C, the housings 72 can be melted together to form the sterilizing bracelet assembly 70 with a ring type.

When the cover 723 is opened and the housing 72 is squeezed, the nozzle 720 sprays some liquid. Furthermore, each of the housings 720 has the first accommodation room 721, so that different first accommodation rooms 721 store different liquids. In another word, some of the first accommodation rooms 721 keep alcohol, but some for-hand lotion, face wash, dry-cleaning hand lotion, etc.

Additionally, the sterilizing bracelet assembly 70 is a one-time consumption or a disposable consumer goods. Frankly speaking, the housing 72 can be squeezed to damage in order to obtain alcohol or hand lotion. That is, the housing 72 may be composed of a material with low mechanical strength for easily damaging the housing 72.

Please refer to Fig. 11, which illustrates a perspective view of another aspect of the sterilizing bracelet assembly 70 of the present invention. As shown in Fig. 10A, the aspect of the sterilizing bracelet assembly 70 is similar to a string of Buddha beads. However, the persons who skill in the art are able to develop a cylindrical bracelet as another aspect of the sterilizing bracelet assembly 70 for easily carrying on.

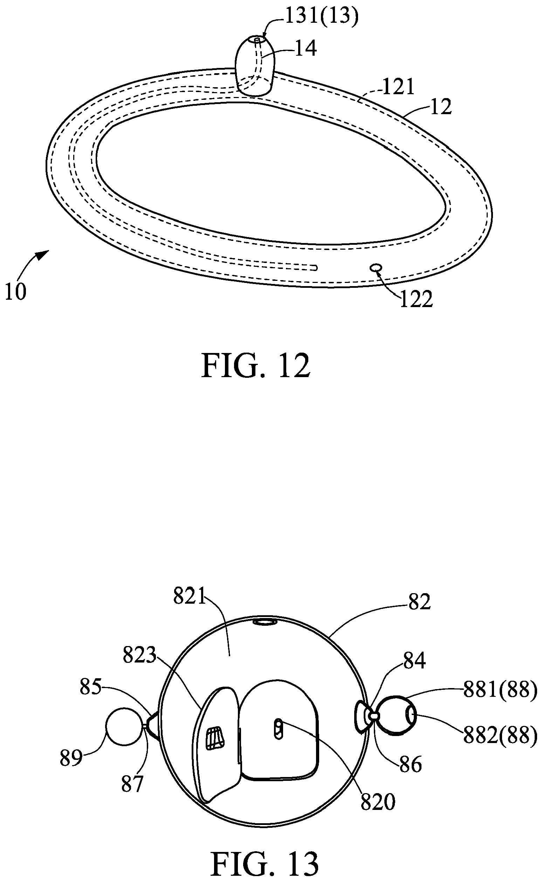

With reference to Fig. 12, which illustrates a perspective view of another aspect of the sterilizing bracelet assembly 10 of the present invention. Compared with Fig. 1B, the sterilizing bracelet assembly 10 in Fig. 12 not only has the housing 12 and the switch 13, but also an extending tube 14, which is extended from the nozzle 131 to an internal of the housing 12. Squeezing the switch 13 is to compress the air pressure in the housing 12 in order to produce an air pressure difference between the internal of the housing 12 and the surrounding. Continuously, the liquid as alcohol, dry-cleaning hand lotion, hand lotion, etc. in the housing 12 accompanies the compressed air to go outside along the extending tube 14 and the nozzle 131. Additionally, the persons who skill in the art may change the design of the switch 13 to the designs of spray nozzles in general market.

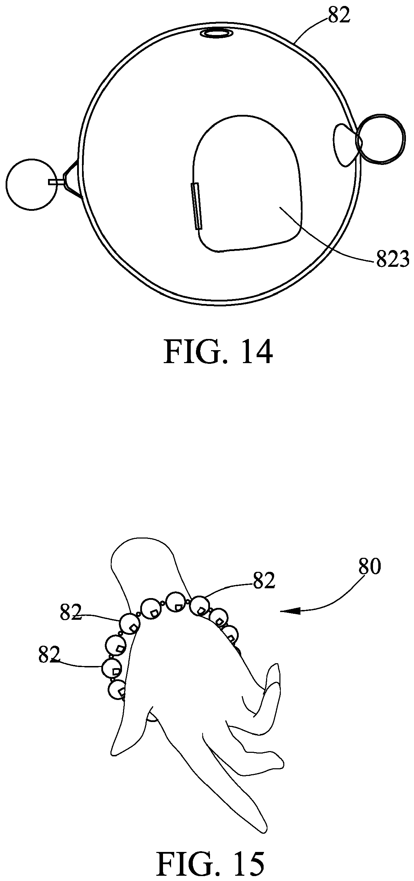

Referring to Fig. 13, Fig. 14 and Fig. 15, which illustrate schematic views of a housing 82 under different applications and a perspective view of a sterilizing bracelet assembly 80 of an eighth preferred embodiment of the present invention. The sterilizing bracelet assembly 80 has a plurality of housings 82. Each of the housing 82 has a nozzle 820, a first accommodation room 821, a cover 823, and two retaining structures 84 and 85. The nozzle 820 is disposed on the surface of the housing 82, and the cover 823 is to cover or uncover the nozzle 820, as shown in Fig. 13 and Fig. 14. The two retaining structures 84 and 85 are disposed on the surface of the housing 82 correspondingly. Each of the two retaining structures 84 and 85 is buckled up with each of two snap rings 86 and 87. An outer ball 88 with a harder housing 881 and a hole 882 is buckled up with the snap ring 86. As it can be seen, a position where the outer ball 88 is connected with the snap ring 86 is corresponding to the hole 882. An inner ball 89 is connected with the snap ring 87. The hole 882 of the outer ball 88 can be female-connected with the inner ball 89 of the other adjacent housing 82. To complete such connection, the inner ball 89 of the other adjacent housing 82 can be inserted into the hole 882 of the outer ball 88 via a squeezing force. Hence, the plurality of the housings 82 are connected with each other so as to form the sterilizing bracelet assembly 80 that is worn on a wrist, as shown in Fig. 15.

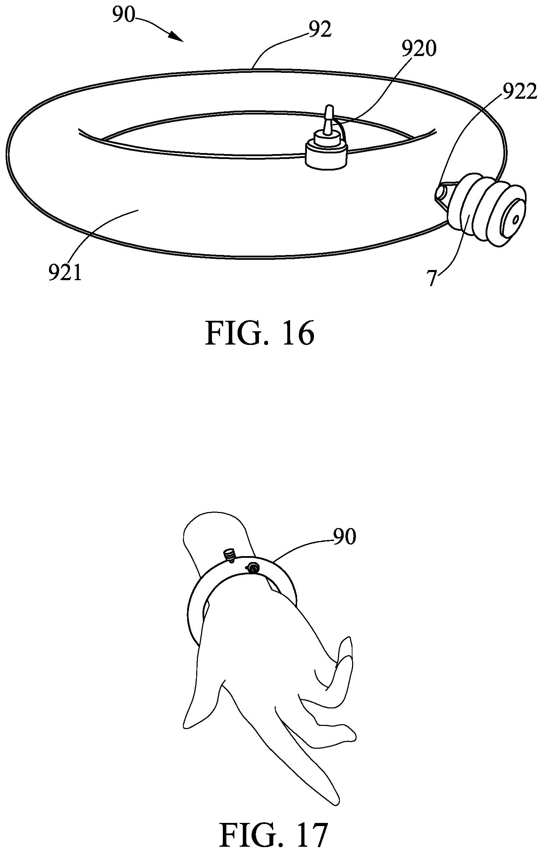

With reference to Fig. 16 and Fig. 17, which illustrate a schematic view of a sterilizing bracelet assembly 90 of a nineth preferred embodiment of the present invention and a perspective view of the sterilizing bracelet assembly 90 under an application. The sterilizing bracelet assembly 90 with a loop type has a housing 92 with a first accommodation room 921, an injection hole 922 and a nozzle 920, and is form as a loop. An external supplement machine 7 is able to inject liquid into the first accommodation room 921 by means of the injection hole 922. Once the sterilizing bracelet assembly 90 is squeezed and the nozzle 920 is uncovered, the liquid may be sprayed out to the surrounding. Therefore, the liquid as alcohol can be practically applied when the sterilizing bracelet assembly 90 is worn on a wrist, as shown in Fig. 17.

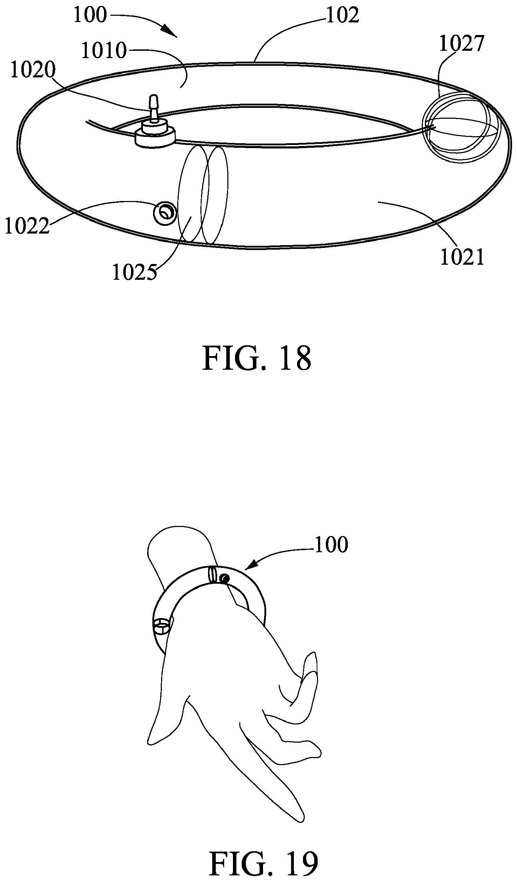

Please refer to Fig. 18 and Fig. 19, which illustrate a schematic view of a sterilizing bracelet assembly 100 of a nineth preferred embodiment of the present invention and a perspective view of the sterilizing bracelet assembly 100 under an application. The sterilizing bracelet assembly 100 with a loop type has a housing 102, an injection hole 1022, a nozzle 1020, a partition 1025, and a blocking structure 1027, wherein the partition 1025 and the blocking structure 1027 divide the inner space of the housing 102 into two rooms, which are a first accommodation room 1021 and a second accommodation room 1010. Liquid can be injected into the second accommodation room 1010 via the injection hole 1022, and therefore squeezing the external surface of the first accommodation room 1021 of the housing 102 may push the blocking structure 1027 moving toward the injection hole 1022 in order to compress the second accommodation room 1010, so that the liquid in the second accommodation room 1010 may be injected to the surrounding by means of the nozzle 1020. For the loop type, the sterilizing bracelet assembly 100 is conveniently worn on a wrist for carrying, as shown in Fig. 19.

To conclude, the sterilizing bracelet assembly of the present invention is very convenient to wrap around the wrist of a human being. So it is useful for modern people to go out and carry and clean their hands.

Although the invention has been disclosed and illustrated with reference to particular embodiments, the principles involved are susceptible for use in numerous other embodiments that will be apparent to persons skilled in the art. This invention is, therefore, to be limited only as indicated by the scope of the appended claims

## Claims

1. A sterilizing bracelet assembly comprising:
a housing, having at least a first accommodation room that is to accommodate liquid; and
at least one switch, connected to the housing and having a nozzle;
wherein some of the liquid is ejected from the nozzle after the switch is squeezed.

2. The sterilizing bracelet assembly according to claim 1 further comprising an electric ring, which comprises:
a touch switch; and
a first Bluetooth module, sending an activating signal after the touch switch is touched;
wherein the switch comprises a second Bluetooth module that is to receive the activating signal, some of the liquid being ejected out from the nozzle after the activating signal is received.

3. The sterilizing bracelet assembly according to claim 1 further comprising a smart watch, which comprises:
a first Bluetooth module, sending an activating signal after the smart watch receives a sound signal;
wherein the switch comprises a second Bluetooth module that is to receive the activating signal, some of the liquid being ejected out from the nozzle after the activating signal is received.

4. The sterilizing bracelet assembly according to claim 3, wherein the smart watch further comprises a display screen for displaying a remaining amount of the liquid.

5. The sterilizing bracelet assembly according to claim 1, wherein the switch comprises:
a valve, being between the first accommodation room and the nozzle, the valve being at an open state when the switch is vertical to the housing, the valve being at a close state when the switch is parallel to the housing.

6. The sterilizing bracelet assembly according to claim 1, wherein the number of the switch is multiple and the number of the first accommodation room is multiple, each of the plurality of switches being corresponding to each of the plurality of first accommodation rooms.

7. The sterilizing bracelet assembly according to claim 1 further comprising two magnets, one of the magnets being disposed at one end of the housing, another being at another end of the housing.

8. The sterilizing bracelet assembly according to claim 1, wherein the liquid is injected into the first accommodation room from an external supplement machine.

9. The sterilizing bracelet assembly according to claim 1 further comprising an extending tube, which is extended from the nozzle to an internal of the housing.

10. A sterilizing bracelet assembly comprising:
a housing, having two first accommodation rooms that are to accommodate liquid;
a supplement box, having two second accommodation rooms that are to accommodate the liquid; and
two switches, connected to the supplement box, each of the two switches having a nozzle and being corresponding to each of the two second accommodation rooms;
wherein one of the first accommodation rooms is connected to one of the second accommodation rooms, and the other first accommodation room is connected to the other second accommodation room, some of the liquid being ejected from the nozzle after the switch is squeezed.

11. The sterilizing bracelet assembly according to claim 1 or claim 10, wherein the switch is selected from the group consisted of: pneumatic switch and electronic switch.

12. A sterilizing bracelet assembly comprising:
a plurality of housings, each of the housings linked another adjacent housing to form a loop, each housing having a first accommodation room that is to accommodate liquid.

13. The sterilizing bracelet assembly according to claim 13, wherein each of the housings comprises:
a through hole; and
a connecting line, which is connected to each of the housings in series by means of the through holes.

14. The sterilizing bracelet assembly according to claim 13, wherein the housing further comprising:
a nozzle, disposed on a surface of the housing; and
a cover, blocking the nozzle;
wherein some of the liquid is ejected from the nozzle after the housing is squeezed.

15. The sterilizing bracelet assembly according to claim 13, wherein the housing further comprising:
a nozzle, disposed on a surface of the housing; and
a cover, blocking the nozzle;
wherein some of the liquid is ejected from the nozzle after the housing is squeezed.
